# EUROPEAN PATENT APPLICATION

(11) **EP 4 464 168 A1**
(43) Date of publication of application: **20.11.2024**
(21) Application number: 22883025.3
(22) Date of filing: 17.10.2022
(51) Int. Cl.: A23K 50/90, A23K 20/10, A23K 10/30

(54) **SUPPLEMENTAL FUNGUS-BASED FEED FOR BEES AND USE OF SAME**

(30) Priority: 18.10.2021 ES 202130969
(71) Applicant: Hifas Veterinary, S.L., 36154 Pontevedra (ES)
(72) Inventor: FERNÁNDEZ DE ANA PORTELA, Catalina, 36154 Pontevedra (ES); SINDE STOMPEL, Esteban, 36154 Pontevedra (ES); LÓPEZ DE GUEREÑU NODAR, Paloma, 36003 Pontevedra (ES)
(74) Representative: Álvarez Flores, Alberto
(86) International application number: PCT/ES2022/070653
(87) International publication number: WO 2023/067215

(57) **Abstract**

A fungal food supplement for bees, comprising carpophore of the species Ganoderma lucidum, Coriolus versicolor, Polyporus umbellatus, Pleurotus ostreatus and/or Cordyceps militaris, preferably, each in a proportion of 18-22% by weight.

The food may also comprise mycelium extract of *Hericium erinaceus.*

This food is especially used to stimulate brood production in bees.

## Description

### TECHNICAL FIELD

The present invention relates to a food supplement for bees having active ingredients of fungal origin, which helps to reduce stress in these insects and increase brood production, especially in autumn.

The scope of application of this invention would be the environment, bee-keeping and pest control.

### BACKGROUND ART

Application US2020376055 describes the use of fungi to improve bee populations. It discloses the use of fungal species such as *Ganoderma lucidum, Trametes versicolor* (synonym *Coriolus versicolor*) and/or combinations thereof, in addition to *Pleurotus ostreatus* and species of the genus *Polyporus* and *Cordyceps.* It describes the increase in resistance to parasites, in addition to their antiviral, antibacterial and/or antifungal activity, but the examples only demonstrate the effectiveness of the mycelium and extract, and do not provide data on carpophore effectiveness.

### BRIEF EXPLANATION OF THE INVENTION

The terms of the present invention are used with the following meanings:
"Mycelium" is the vegetative part of the fungus, which consists of a network of filaments called hyphae.
"Carpophore" is the reproductive part of the fungus, also called mushroom or fruiting body.

The invention consists of a food supplement for bees having active ingredients of fungal origin, which helps to increase brood production in these insects, especially in autumn.

Although it has been described that the use of the fungal mycelium has certain applications in bees, such as resistance to parasites, none provides solutions for strengthening the colony and increasing brood production, which is a key parameter in the colony's survival. We detected that, by using carpophore powder of the species *Ganoderma lucidum, Cordyceps militaris, Coriolus versicolor, Polyporus umbellatus* and *Pleurotus ostreatus,* increased brood production is achieved during the autumn months. Surprisingly, the effect of the carpophore is significantly greater than the effect of the mycelium.

The aim of the patent is to protect the composition of an oral food supplement intended for bees. The active ingredients are of fungal origin based on carpophore, preferably powdered, of the species *Ganoderma lucidum, Cordyceps militaris, Coriolus versicolor, Polyporus umbellatus* and *Pleurotus ostreatus,* preferably in the same proportion. These carpophore-based ingredients are preferred to the use of the mycelium of these species due to having reported conclusive and significantly superior results in terms of increased brood production. Each of the ingredients of fungal origin based on carpophore of the species *Ganoderma lucidum, Cordyceps militaris, Coriolus versicolor, Polyporus umbellatus* and *Pleurotus ostreatus* is advantageous in itself. The combination of all the fungal species, for example in a proportion of 18-22% by weight, preferably 20%, is preferred before the addition of any other additive. An example of an additive is mycelial extract of Hericium erinaceus.

It is a product used to stimulate laying, preferably before hibernation and first flowering, which is helpful when transporting hives and in other situations that may cause stress in the colony (adverse weather conditions; presence of invasive species; bacterial, viral and parasitic diseases; etc.).

Other variants will be discussed in other points of the description.

### DESCRIPTION OF THE FIGURES

A figure is provided to facilitate the understanding of the invention:

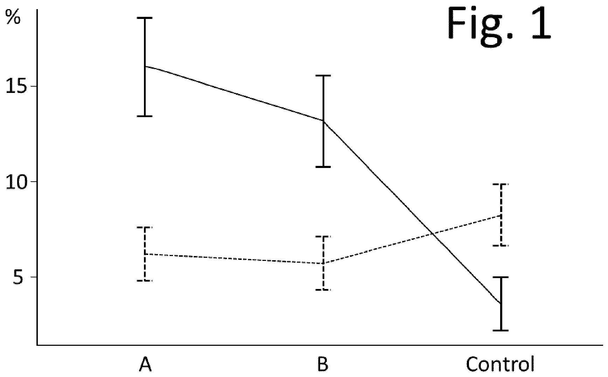

Figure 1: Comparative of brood yield (en percentage) using carpophore (A), mycelium (B) and the control group.

### EMBODIMENTS OF THE INVENTION

Following is a brief descriptionof an embodiment of the invention, by way of illustrative and non-limiting example thereof.

The aim of the patent is to protect the composition of an oral food supplement intended for bees. The active ingredients are ingredients of fungal origin based on carpophore powder of the species *Ganoderma lucidum, Cordyceps militaris, Coriolus versicolor, Polyporus umbellatus* and *Pleurotus ostreatus.*

The composition may contain sucrose, dextrose, fructose, soy flour, brewer's yeast and/or other nutritional or attractive compounds for bees. They may also contain preservatives such as potassium sorbate.

The supplement is administered orally, based on the needs and seasonal activity of the hive. The availability of fresh water and nectar or, failing that, a syrup composed of easily assimilated sugars must be guaranteed. In Langstroth hives, the product is placed on the brood chamber, in a place where it is easily accessible by the nurse bees. In Layens hives, it is placed preferably between the brood frames.

### Example 1

The objective of example 1 is to evaluate the effect of supplementing the liquid feed of bees with a solid diet in the form of a cake containing a dehydrated fungal carpophore powder complex to strengthen the immune system of bees.

Two supplementation formulas are used:
Formula 1: Fructose, sucrose, dextrose, soy flour, brewer's yeast and water.
Formula 2: Formula 1 including 2.5% of dehydrated ground carpophore of the species *Ganoderma lucidum, Coriolus versicolor, Polyporus umbellatus, Pleurotus ostreatus* and *Cordyceps militaris* in a mixture in equal proportion (20% u.c.).

The beehives are supplemented for 3 months from October to December. Consumption ranges between 40.24 g/week of formula 1 and 27.06 g/day of formula 2, of which 0.66 g/week correspond to the carpophore mixture of the fungi.

Brood yield is analysed, which is an indicator of the reproductive capacity of a beehive to produce broods and therefore increase the colony. It is measured by visually estimating the percentage of occupancy of hive brood frame cells by eggs/larvae (for example: 25%, 50%, 75%).

The average brood percentage of formula 1 is 5.25%, whereas the average brood percentage of formula 2 is 13.65%. Brood yield is significantly higher in hives where formula 2, which contains fungi, is administered, compared to formula 1.

### Example 2

The objective of example 2 is to evaluate the effect of supplementing the liquid feed of bees with a solid diet in the form of a cake containing a dehydrated fungal mycelium powder complex to strengthen the immune system of bees.

Two supplementation formulas are used:
Formula 1: Fructose, sucrose, dextrose, soy flour, brewer's yeast and water.
Formula 2: Formula 1 including 2.5% of dehydrated ground mycelium of the species *Ganoderma lucidum, Coriolus versicolor, Polyporus umbellatus, Pleurotus ostreatus* and *Cordyceps militaris* in a mixture in equal proportion (20% u.c.).

The supplementation is carried out over a three-month period, from November to January. Consumption ranges between 90.00 g/week of formula 1 and 90.68 g/week of formula 2. It corresponds approximately to 2.22 g/week per hive of fungi of formula 2.

Brood yield is analysed, which is an indicator of the reproductive capacity of a beehive to produce broods and therefore increase the colony. It is measured by visually estimating the occupancy percentage of hive brood frame cells by eggs/larvae (for example: 25%, 50%, 75%).

The average brood percentage of formula 1 (control group) is 5.60%, while the average brood percentage of formula 2 is 5.05%. There are no significant differences between the average brood percentage of the control group (formula 1) and of the group treated with mycelium (formula 2).

The average brood percentage of the food supplement containing carpophore-based ingredients (formula 2 of example 1) is 13.65%, whereas the average brood percentage using mycelium-based ingredients (formula 2 of example 2) is 5.05%. The effectiveness of carpophore is significantly superior to the effectiveness of mycelium in brood production. Significant differences with respect to the control group were only observed in the food supplemented with carpophore-based ingredients. Surprisingly, there is a twofold increase in brood percentage. The comparison of the results in both tests is shown in figure 1. The carpophore example is shown with a solid line and the mycelium example 2 with a dashed line.

## Claims

1. A fungal food supplement for bees, comprising carpophore of the species *Ganoderma lucidum, Coriolus versicolor, Polyporus umbellatus, Pleurotus ostreatus* and/or *Cordyceps militaris.*

2. The fungal food supplement for bees, according to claim 1, **characterised in that** it comprises carpophore of the species *Ganoderma lucidum, Coriolus versicolor, Polyporus umbellatus, Pleurotus ostreatus* and *Cordyceps militaris, each in a proportion of 18-22% by weight.*

3. The fungal food supplement for bees, according to claim 1, **characterised in that** the carpophore is powdered.

4. The fungal food supplement for bees, according to claim 1, **characterised in that** it also comprises mycelium extract of *Hericium erinaceus.*

5. The fungal food supplement for bees, according to claim 1, **characterised in that** it comprises fructose, sucrose, dextrose, soya flour, beer yeast and water.

6. Use of a fungal food supplement for bees, according to any of claims 1 to 5, for increasing brood production in bees.
